(19) Europäisches Patentamt / European Patent Office / Office européen des brevets

(11) **EP 2 998 735 A1**

(12) **EUROPEAN PATENT APPLICATION**

(43) Date of publication:
**23.03.2016 Bulletin 2016/12**

(51) Int Cl.:
**G01N 29/24** *(2006.01)*     **A61B 5/00** *(2006.01)*
**G01S 15/89** *(2006.01)*     **G01N 21/17** *(2006.01)*

(21) Application number: **15180502.5**

(22) Date of filing: **11.08.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **18.08.2014 JP 2014165874**

(71) Applicant: **PreXion Corporation**
**Chiyoda-ku**
**Tokyo 101-0041 (JP)**

(72) Inventors:
• **NAKATSUKA, Hitoshi**
**Chiyoda-ku Tokyo 101-0041 (JP)**
• **AGANO, Toshitaka**
**Chiyoda-ku Tokyo 101-0041 (JP)**

(74) Representative: **Grünecker Patent- und Rechtsanwälte**
**PartG mbB**
**Leopoldstraße 4**
**80802 München (DE)**

(54) **PHOTOACOUSTIC IMAGER**

(57)     This photoacoustic imager (100, 200, 300, 400, 500, 600, 700) includes a light source portion (11a, 411a, 411b, 511a, 611a), a detection portion (13), an amplification portion (22, 222, 322, 422, 702) and an imaging portion (23), and the amplification portion is so configured that an acoustic gain which is a gain of the amplification portion for amplifying a detection signal resulting from an acoustic wave is larger than an ultrasonic gain which is a gain of the amplification portion for amplifying a detection signal resulting from an ultrasonic wave.

FIG.1   FIRST EMBODIMENT

**EP 2 998 735 A1**

## Description

BACKGROUND OF THE INVENTION

Field of the Invention

The present invention relates to a photoacoustic imager, and more particularly, it relates to a photoacoustic imager including a detection portion detecting an acoustic wave resulting from light applied to a specimen and an ultrasonic wave applied to and reflected by the specimen.

Description of the Background Art

A photoacoustic imager including a detection portion detecting an acoustic wave resulting from light applied to a specimen and an ultrasonic wave applied to and reflected by the specimen is known in general, as disclosed in Japanese Patent Laying-Open No. 2012-196308, for example.

The aforementioned Japanese Patent Laying-Open No. 2012-196308 discloses a photoacoustic image generator including an ultrasonic probe detecting a photoacoustic signal resulting from a laser beam applied to a specimen and a reflected acoustic signal applied to and reflected by the specimen. This photoacoustic image generator is provided with a light source unit including a Q-switched pulsed laser beam source, the ultrasonic probe and a signal processing portion. The photoacoustic image generator is configured to apply a laser beam from the light source unit to the specimen, to apply an ultrasonic wave from the ultrasonic probe to the specimen and to detect an acoustic signal and a reflected acoustic signal generated by the specimen with the ultrasonic probe. The signal processing portion is configured to generate a photoacoustic image and an ultrasonic image on the basis of the acoustic signal and the reflected acoustic signal detected by the ultrasonic probe.

In order to miniaturize the light source unit of the photoacoustic image generator according to the aforementioned Japanese Patent Laying-Open No. 2012-196308, a structure of providing a light-emitting diode element (a light-emitting element) as a light source is conceivable, for example. In general, however, the light-emitting diode element has a smaller quantity of light as compared with the Q-switched pulsed laser beam source, and hence the intensity of the acoustic signal is conceivably reduced when the light-emitting diode element is employed. Therefore, a structure of providing an amplifier on the aforementioned photoacoustic image generator for amplifying the intensity of a detection signal detected by the ultrasonic probe (a detection portion) with the amplifier is conceivable. In this case, the intensity of the acoustic signal can be set to a proper magnitude by amplifying the intensity of the detection signal with the amplifier, while the intensity of the reflected acoustic signal may conceivably be disadvantageously excessively increased. In other words, it is conceivable that the amplifier having a gain for amplifying the acoustic signal (an acoustic wave) having relatively small intensity so amplifies the intensity of the reflected acoustic signal (an ultrasonic wave) that the intensity of the signal (the detection signal) input in the signal processing portion (an imaging portion) is saturated. When the quantity of the light applied to the specimen is small, therefore, it may conceivably be difficult to properly image both of the acoustic wave and the ultrasonic wave.

SUMMARY OF THE INVENTION

The present invention has been proposed in order to solve the aforementioned problem, and an object of the present invention is to provide a photoacoustic imager capable of preventing difficulty in proper imaging of an acoustic wave and an ultrasonic wave also when the quantity of light applied to a specimen is small.

In order to attain the aforementioned object, a photoacoustic imager according to an aspect of the present invention includes a light-emitting element applying light to a specimen, a detection portion configured to apply an ultrasonic wave to the specimen and to generate a detection signal by detecting the ultrasonic wave reflected by a detection object in the specimen and an acoustic wave generated by the detection object in the specimen absorbing the light applied from the light-emitting element to the specimen, an imaging portion configured to perform imaging on the basis of the detection signal and an amplification portion configured to amplify the detection signal generated by the detection portion and to transmit the detection signal to the imaging portion, while the amplification portion is so configured that an acoustic gain which is a gain of the amplification portion for amplifying the detection signal resulting from the acoustic wave is larger than an ultrasonic gain which is a gain of the amplification portion for amplifying the detection signal resulting from the ultrasonic wave.

In the photoacoustic imager according to the aspect of the present invention, as hereinabove described, the amplification portion is so configured that the acoustic gain which is the gain of the amplification portion for amplifying the detection signal resulting from the acoustic wave is larger than the ultrasonic gain which is the gain of the amplification portion for amplifying the detection signal resulting from the ultrasonic wave. Thus, the photoacoustic imager can prevent saturation (excess enlargement) of the detection signal for the ultrasonic wave in the imaging portion while preventing the detection signal for the acoustic wave from weakening also when employing the light-emitting element having a relatively small quantity of light. Consequently, the photoacoustic imager can prevent difficulty in proper imaging of the acoustic wave and the ultrasonic wave also when the quantity of the light applied to the specimen is small.

In the photoacoustic imager according to the aforementioned aspect, the amplification portion is preferably configured to switch the acoustic gain and the ultrasonic gain, and the imaging portion is preferably configured not to acquire the detection signal in a signal non-acquiring period including a time for switching the acoustic gain and the ultrasonic gain. At (during) the time for switching the acoustic gain and the ultrasonic gain, the amplification portion may conceivably erroneously amplify the detection signal resulting from the acoustic wave with the ultrasonic gain or erroneously amplify the detection signal resulting from the ultrasonic wave with the acoustic gain. In this case, it is conceivable that the detection signal for the acoustic wave is weakened or the detection signal for the ultrasonic wave is saturated in the imaging portion. When configured to be provided with the signal non-acquiring period including the time for switching the acoustic gain and the ultrasonic gain as in the present invention, the photoacoustic imager can more reliably prevent the detection signal for the ultrasonic wave from saturation in the imaging portion while preventing the detection signal for the acoustic wave from weakening.

In this case, the amplification portion is preferably configured to switch the acoustic gain and the ultrasonic gain after switching the imaging portion from a state of acquiring the detection signal to a state of not acquiring the detection signal. According to this structure, the amplification portion can be more reliably prevented from erroneously amplifying the detection signal resulting from the acoustic wave with the ultrasonic gain or erroneously amplifying the detection signal resulting from the ultrasonic wave with the acoustic gain at (during) the time for switching the acoustic gain and the ultrasonic gain.

In the aforementioned photoacoustic imager including the amplification portion switching the acoustic gain and the ultrasonic gain, the amplification portion preferably includes a coupling capacitor for acquiring the detection signal, and the signal non-acquiring period is preferably larger than a time constant calculated with the capacitance of the coupling capacitor and the input impedance of the amplification portion. The length of a time (a settling time) required for settling a factor, such as a reflected wave or a crosstalk caused during or immediately after switching of the acoustic gain and the ultrasonic gain, inhibiting the amplification portion from detecting the acoustic wave and the ultrasonic wave is related to the magnitudes of the capacitance of the coupling capacitor of the amplification portion and the input impedance of the amplification portion. When the photoacoustic imager is so configured that the signal non-acquiring period is larger than the time constant calculated with the capacitance of the coupling capacitor and the input impedance of the amplification portion as in the present invention, therefore, the imaging portion acquires no detection signal during the time (the settling time) required for settling the factor inhibiting the amplification portion from detecting the acoustic wave and the ultrasonic wave, whereby the photoacoustic imager can be prevented from influence by the factor inhibiting the amplification portion from detecting the acoustic wave and the ultrasonic wave. Consequently, the photoacoustic imager can further reliably prevent the detection signal for the ultrasonic wave from saturation in the imaging portion while preventing the detection signal for the acoustic wave from weakening.

In the aforementioned photoacoustic imager including the amplification portion switching the acoustic gain and the ultrasonic gain, the signal non-acquiring period is preferably larger than a time required for settling a factor inhibiting the amplification portion from detecting the acoustic wave. According to this structure, the imaging portion does not enter a state of acquiring the detection signal during the settling time required for settling the factor inhibiting the amplification portion from detecting the acoustic wave and the ultrasonic wave, whereby the photoacoustic imager can prevent inhibition of the detection of the acoustic wave and the ultrasonic wave. Consequently, the photoacoustic imager can prevent the detection signal for the acoustic wave from weakening and can also prevent the detection signal for the ultrasonic wave from saturation in the imaging portion.

In the aforementioned photoacoustic imager including the amplification portion switching the acoustic gain and the ultrasonic gain, the signal non-acquiring period is preferably smaller than a period for acquiring the acoustic wave by applying the light from the light source portion to the specimen. According to this structure, the signal non-acquiring period is smaller than the period for acquiring the acoustic wave, whereby the period for acquiring the acoustic wave can be prevented from decreasing also when the signal non-acquiring period is provided.

In the photoacoustic imager according to the aforementioned aspect, a clamp circuit is preferably provided on an output side of the amplification portion. When the detection signal is saturated in the imaging portion in general, there arises a period when the imaging portion cannot normally acquire the detection signal after the saturation of the detection signal. When the photoacoustic imager is so configured that the clamp circuit is provided on the output side of the amplification portion (on an input side of the imaging portion) as in the present invention, therefore, the photoacoustic imager can restrict the intensity (voltage) of the detection signal output from the amplification portion (input in the imaging portion), whereby the same can prevent the detection signal from saturation in the imaging portion also when a relatively large detection signal is input in the amplification portion having the acoustic gain, for example. Consequently, the photoacoustic imager can quickly image the acoustic wave and the ultrasonic wave with the imaging portion also when a relatively large detection signal is input in the amplification portion having the acoustic gain.

In this case, the clamp circuit preferably includes a first diode having an anode connected to the output side of the amplification portion and a cathode connected to an external power source portion and a second diode having a grounded anode and a cathode connected to the output side of the amplification portion. According to this structure, current flows from the amplification portion to the external power source portion when the voltage on the output side of the amplification portion exceeds the voltage value of the external power source portion, whereby the photoacoustic imager can prevent the detection signal from overshooting (saturation) with respect to the input side of the imaging portion. When the voltage value on the output side of the amplification portion is reduced below zero, on the other hand, the photoacoustic imager can prevent the detection signal from undershooting with respect to the input side of the imaging portion.

In the photoacoustic imager according to the aforementioned aspect, the light-emitting element preferably includes a first light-emitting element emitting light having a first wavelength and a second light-emitting element emitting light having a second wavelength, and the amplification portion is preferably so configured that a first acoustic gain which is a gain of the amplification portion for amplifying the detection signal resulting from the acoustic wave generated due to the light from the first light-emitting element and a second acoustic gain which is a gain of the amplification portion for amplifying the detection signal resulting from the acoustic wave generated due to the light from the second light-emitting element have values different from each other and also so configured that the first acoustic gain and the second acoustic gain are larger than the ultrasonic gain. According to this structure, the photoacoustic imager can prevent the detection signal resulting from the acoustic wave generated due to the light from the second light-emitting element and the detection signal resulting from the ultrasonic wave from excess enlargement while preventing the detection signal resulting from the acoustic wave generated due to the light from the first light-emitting element from weakening by rendering the first acoustic gain larger than the second acoustic gain when the specimen has a relatively small absorption coefficient for the light having the first wavelength and a relatively large absorption coefficient for the light having the second wavelength, for example.

In the photoacoustic imager according to the aforementioned aspect, the light-emitting element is preferably constituted of a light-emitting diode element. According to this structure, the light-emitting diode element is lower in directivity as compared with a light-emitting element emitting a laser beam, and hence a light emission range remains relatively unchanged also when misregistration takes place. Therefore, the photoacoustic imager requires neither precise alignment (registration) of optical members nor an optical platen or a strong housing for preventing characteristic fluctuation resulting from vibration of an optical system, dissimilarly to a case of employing a light-emitting element emitting a laser beam. Consequently, the photoacoustic imager can be prevented from size increase and complication in structure due to the nonrequirement for precise alignment of optical members and an optical platen or a strong housing. Further, the light-emitting diode element has a smaller quantity of light as compared with a light-emitting element emitting a laser beam or the like. When the amplification portion is so configured that the acoustic gain is larger than the ultrasonic gain as in the present invention, therefore, the photoacoustic imager can further effectively prevent difficulty in proper imaging of the acoustic wave and the ultrasonic wave.

In the photoacoustic imager according to the aforementioned aspect, the light-emitting element is preferably constituted of a semiconductor laser element. According to this structure, the semiconductor laser element can apply a laser beam relatively higher in directivity as compared with a light-emitting diode element to the specimen, whereby the photoacoustic imager can reliably apply most part of the light from the semiconductor laser element to the specimen.

In the photoacoustic imager according to the aforementioned aspect, the light-emitting element is preferably constituted of an organic light-emitting diode element. According to this structure, the light source portion including the organic light-emitting diode element can be easily miniaturized by employing the organic light-emitting diode element easily reducible in thickness.

In the photoacoustic imager according to the aforementioned aspect, the light-emitting element is preferably configured to emit pulsed light having a wavelength in the infrared region. According to this structure, the light having the wavelength in the infrared region can relatively easily penetrate a human body, whereby the photoacoustic imager can deliver the light from the light source portion to a deeper portion of the specimen when the specimen is prepared from a human body.

In the photoacoustic imager according to the aforementioned aspect, the amplification portion preferably includes a first amplifier having a first prescribed gain and a second amplifier having a second prescribed gain, the acoustic gain is preferably a gain obtained by synthesizing the gain of the first amplifier and the gain of the second amplifier, and the ultrasonic gain is preferably the gain of the second amplifier. According to this structure, the photoacoustic imager can be easily so configured that the acoustic gain is larger than the ultrasonic gain.

In the photoacoustic imager according to the aforementioned aspect, the amplification portion preferably includes a switch portion for switching a state corresponding to the gain of the first amplifier and the gain of the second amplifier and a state corresponding to the gain of the second amplifier. According to this structure, the photoacoustic imager can easily switch the acoustic gain and the ultrasonic gain with the switch portion.

In the photoacoustic imager according to the aforementioned aspect, the amplification portion preferably includes at least a programmable gain amplifier in either the programmable gain amplifier or an attenuator.

According to this structure, the programmable gain amplifier and the attenuator can control gains with an analog signal, whereby the photoacoustic imager can suppress influence by an electromagnetic wave or the like (noise) resulting from a control signal as compared with a case of controlling gains of a plurality of amplifiers by supplying a digital signal to the switch portion.

In this case, the photoacoustic imager preferably further includes a control portion transmitting a gain control signal consisting of an analog signal to the amplification portion, and the amplification portion is preferably configured to switch the acoustic gain and the ultrasonic gain with at least the programmable gain amplifier in either the programmable gain amplifier or the attenuator. According to this structure, the photoacoustic imager can switch the acoustic gain and the ultrasonic gain with the gain control signal consisting of the analog signal, whereby the same can easily switch the acoustic gain and the ultrasonic gain while suppressing influence by an electromagnetic wave or the like (noise) resulting from the control signal.

The photoacoustic imager according to the aforementioned aspect preferably further includes a probe having the light-emitting element, the detection portion and the amplification portion arranged therein. When a transmitted signal has a relatively small voltage value in general, the photoacoustic imager is easily influenced by an electromagnetic wave or the like (noise) from the exterior. Further, the detection signal output from the detection portion has a relatively small voltage value (on the order of $\mu$V, for example), while voltage output from the amplification portion reaches a relatively large value (on the order of mV to about several V) due to the amplification of the detection signal. Noting this point, the light-emitting element, the detection portion and the amplification portion are arranged in the probe in the photoacoustic imager according to the present invention, whereby the detection signal from the detection portion can be transmitted to the imaging portion from the probe in the state amplified by the amplification portion. Consequently, the photoacoustic imager can suppress influence by an electromagnetic wave or the like (noise) from the exterior when transmitting the detection signal to the imaging portion from the probe.

In the photoacoustic imager according to the aforementioned aspect, the amplification portion is preferably configured to alternately repetitively switch the acoustic gain and the ultrasonic gain. According to this structure, the imaging portion can repetitively acquire the acoustic gain and the ultrasonic gain, whereby the photoacoustic imager can easily image both of the acoustic gain and the ultrasonic gain.

In the photoacoustic imager according to the aforementioned aspect, a period when the amplification portion has the acoustic gain is preferably larger than a period when the amplification portion has the ultrasonic gain. According to this structure, the period for detecting the acoustic wave is larger than the period for detecting the ultrasonic wave also in a case where signal intensity of the acoustic wave is smaller than that of the ultrasonic wave, whereby the photoacoustic imager can prevent the detection signal for the ultrasonic wave from saturation in the imaging portion while preventing the detection signal for the acoustic wave from weakening.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a block diagram showing the overall structure of a photoacoustic imager according to a first embodiment of the present invention;
Fig. 2 is a circuit diagram showing the structure of an amplification portion according to the first embodiment of the present invention;
Fig. 3 is a timing chart for illustrating a receiving accept signal and a gain control signal from a control portion according to the first embodiment of the present invention;
Fig. 4 is a circuit diagram showing the structure of an amplification portion of a photoacoustic imager according to a second embodiment of the present invention;
Fig. 5 is a timing chart for illustrating a receiving accept signal and a gain control signal from a control portion according to the second embodiment of the present invention;
Fig. 6 is a circuit diagram showing the structure of an amplification portion of a photoacoustic imager according to a third embodiment of the present invention;
Fig. 7 is a block diagram showing the overall structure of a photoacoustic imager according to a fourth embodiment of the present invention;
Fig. 8 is a circuit diagram showing the structure of an amplification portion according to the fourth embodiment of the present invention;
Fig. 9 is a timing chart for illustrating a receiving accept signal and a gain control signal from a control portion according to the fourth embodiment of the present invention;
Fig. 10 is a block diagram showing the overall structure of a photoacoustic imager according to a first or second modification of the first embodiment of the present invention; and
Fig. 11 is a block diagram showing the overall structure of a photoacoustic imager according to a third modification of the first embodiment of the present invention.

DESCRIPTION OF THE PREFERRED EMBODIMENTS

Embodiments of the present invention are now described with reference to the drawings.

(First Embodiment)

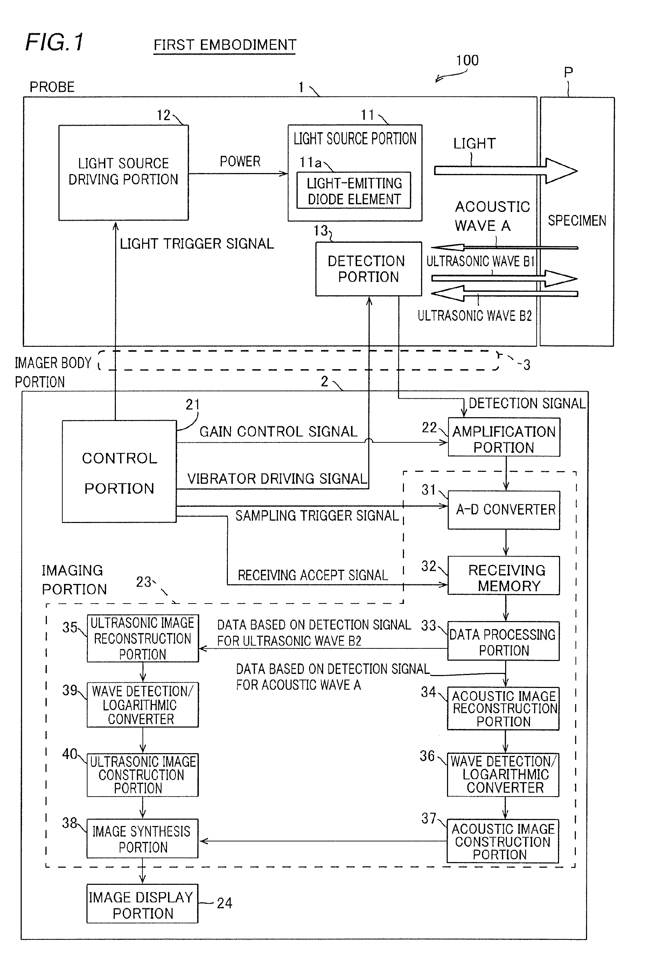

The structure of a photoacoustic imager 100 according to a first embodiment of the present invention is described with reference to Figs. 1 to 3.

The photoacoustic imager 100 according to the first embodiment of the present invention is provided with a probe 1 and an imager body portion 2, as shown in Fig. 1. The photoacoustic imager 100 is also provided with a cable 3 connecting the probe 1 and the imager body portion 2 with each other.

The probe 1 is so configured that the same is grasped by an operator and arranged on a surface of a specimen P (such as a surface of a human body). Further, the probe 1 is configured to be capable of applying light to the specimen P, to detect an acoustic wave $\underline{A}$ and an ultrasonic wave B2, both described later, from the specimen P and to transmit the acoustic wave $\underline{A}$ and the ultrasonic wave B2 to the imager body portion 2 as detection signals through the cable 3.

The imager body portion 2 is configured to process and image the detection signals detected by the probe 1 and to display the imaged acoustic and ultrasonic waves $\underline{A}$ and B2.

The probe 1 is provided with a light source portion 11. The light source portion 11 includes a plurality of light-emitting diode elements 11a. The light-emitting diode elements 11a are configured to be capable of emitting pulsed light having a wavelength in the infrared region (a wavelength of about 600 nm to about 1000 nm, for example, preferably a wavelength of about 850 nm) by being supplied with power from a light source driving portion 12 described later. The light source portion 11 is configured to apply the light emitted by the plurality of light-emitting diode elements 11a to the specimen P. The light-emitting diode elements 11a are examples of the "light-emitting element" in the present invention.

The probe 1 is also provided with the light source driving portion 12. The light source driving portion 12 is configured to acquire power from an external power source (not shown). The light source driving portion 12 is further configured to supply power to the light source portion 11 on the basis of a light trigger signal received from a control portion 21 described later.

A detection object (hemoglobin or the like, for example) in the specimen P absorbs the pulsed light applied from the probe 1 to the specimen P. The detection object (the specimen P) expands and contracts (returns to the original size from an expanding size) in response to the intensity of application (the quantity of absorption) of the pulsed light, thereby generating the acoustic wave $\underline{A}$.

The probe 1 is also provided with a detection portion 13. The detection portion 13 is constituted of a piezoelectric element (lead zirconate titanate (PZT), for example) or the like. The detection portion 13 is configured to vibrate and generate voltage (a detection signal) when acquiring the aforementioned acoustic wave $\underline{A}$. The detection portion 13 is further configured to transmit the acquired detection signal to an amplification portion 22 described later.

The detection portion 13 is also configured to be capable of generating an ultrasonic wave B1 by vibrating at a frequency responsive to a vibrator driving signal received from the control portion 21 provided on the imager body portion 2. A substance, having high acoustic impedance, in the specimen P reflects the ultrasonic wave B1 generated by the detection portion 13. The detection portion 13 detects the ultrasonic wave B2 (resulting from reflection of the ultrasonic wave B1), and is configured to vibrate due to the ultrasonic wave B2. Throughout the specification, the "acoustic wave $\underline{A}$" denotes an ultrasonic wave generated by the detection object in the specimen P absorbing light, and the "ultrasonic wave B2" denotes an ultrasonic wave generated by the detection portion 13 and reflected by the specimen P, for the convenience of illustration.

The detection portion 13 is further configured to transmit a detection signal to the amplification portion 22 also when vibrating due to the ultrasonic wave B2, similarly to the case of vibrating due to the acoustic wave $\underline{A}$.

According to the first embodiment, the imager body portion 2 is provided with the amplification portion 22, which in turn is so configured that an acoustic gain which is a gain of the amplification portion 22 for amplifying the detection signal resulting from the acoustic wave $\underline{A}$ is larger than an ultrasonic gain which is a gain of the amplification portion 22 for amplifying the detection signal resulting from the ultrasonic wave B2. The amplification portion 22 is further configured to be capable of switching the acoustic gain and the ultrasonic gain.

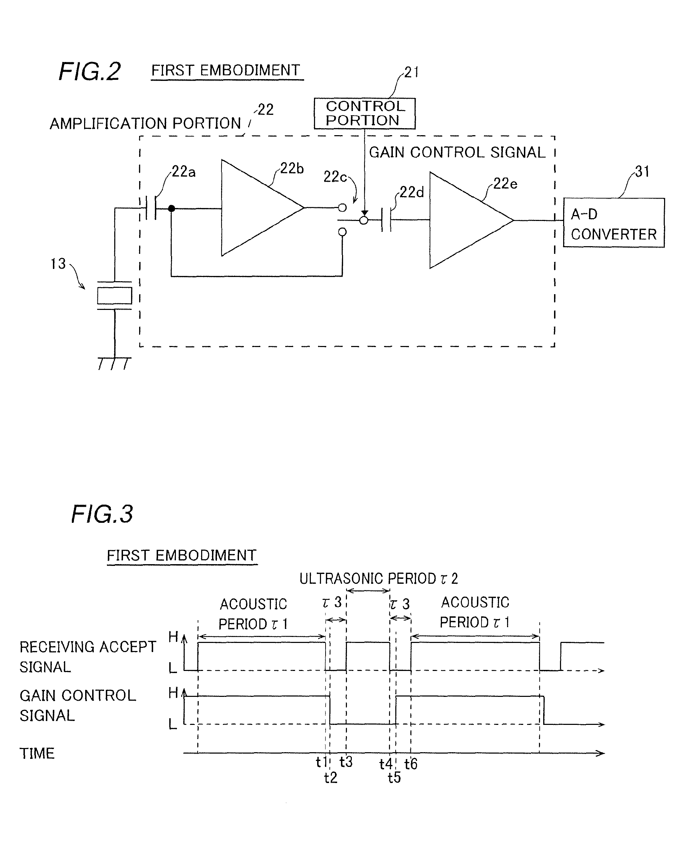

More specifically, the amplification portion 22 is provided with a first coupling capacitor 22a connected to the detection portion 13, as shown in Fig. 2. The first coupling capacitor 22a is configured to transmit a signal of a specific frequency of the detection signal based on the capacitance of the first coupling capacitor 22a, input impedances of first and second amplifiers 22b and 22e described later from the detection portion 13.

The amplification portion 22 is also provided with the first amplifier 22b having an input side connected to the first coupling capacitor 22a and an output side connected to a switch portion 22c described later. The first amplifier 22b is configured to amplify the detection signal acquired through the coupling capacitor 22a and to output the same to the side of the switch portion 22c. For example, the first amplifier 22b is so configured that a gain obtained by synthesizing the gain thereof with the gain of the second amplifier 22e described later is about 80 dB. The gain obtained by synthesizing the gains of the first and second amplifiers 22b and 22e is that of the amplification portion 22 for amplifying the detection signal resulting from the acoustic wave A, and corresponds to the acoustic gain. The gain of the first amplifier 22b is an example of the "first prescribed gain" in the present invention. The gain of the second amplifier 22e is an example of the "second prescribed gain" in the present invention.

The amplification portion 22 is also provided with the switch portion 22c. The switch portion 22c is connected to a second coupling capacitor 22d described later, and configured to be capable of switching the first coupling capacitor 22a or the first amplifier 22b, with which the second coupling capacitor 22d is to be connected, on the basis of a gain control signal received from the control portion 21. For example, the switch portion 22c is configured to set the gain of the amplification portion 22 to about 80 dB by connecting the second coupling capacitor 22d to the first amplifier 22b when the gain control signal is at a high voltage level (see Fig. 3). Further, the switch portion 22c is configured to set the gain of the amplification portion 22 to about 50 dB corresponding to the gain of the second amplifier 22e by connecting the second coupling capacitor 22d to the first coupling capacitor 22a when the gain control signal is at a low voltage level. In other words, the switch portion 22c is configured to switch a state corresponding to the gains of the first and second amplifiers 22b and 22e and a state corresponding to the gain of the second amplifier 22e according to the first embodiment.

The second coupling capacitor 22d connected to the switch portion 22c is connected to an input side of the second amplifier 22e. An output side of the second amplifier 22e is connected to an A-D converter 31 described later, and the second amplifier 22e is configured to amplify a detection signal received from the second coupling capacitor 22d on the input side and to output the amplified detection signal to the side of the A-D converter 31. Further, the second amplifier 22e is configured to have the gain of 50 dB, as described above. In other words, the switch portion 22c is configured to be capable of switching the aforementioned acoustic and ultrasonic gains by being driven on the basis of the gain control signal received from the control portion 21. The gain of the second amplifier 22e is that of the amplification portion 22 for amplifying the detection signal resulting from the ultrasonic wave B2, and corresponds to the ultrasonic gain.

The control portion 21 is configured to perform control of setting the amplification portion 22 to the acoustic gain (about 80 dB) by setting the gain control signal to a high voltage level in a period (an acoustic period) $\tau 1$ for acquiring the acoustic wave A by applying light from the light source portion 11 to the specimen P, as shown in Fig. 3. The control portion 21 is also configured to perform control of setting the amplification portion 22 to the ultrasonic gain (about 50 dB) by setting the gain control signal to a low voltage level in a period (an ultrasonic

period) $\tau 2$ for acquiring the ultrasonic wave B2 by applying the ultrasonic wave B1 from the detection portion 13 to the specimen P. According to the first embodiment, the period (the acoustic period) $\tau 1$ when the amplification portion 22 has the acoustic gain is set to be larger than the period (the ultrasonic period) $\tau 2$ when the amplification portion 22 has the ultrasonic gain. The amplification portion 22 is configured to alternately and repetitively switch the acoustic gain and the ultrasonic gain.

The image body portion 2 is also provided with an imaging portion 23, as shown in Fig. 1.

According to the first embodiment, the imaging portion 23 is configured to acquire no detection signal in a signal non-acquiring period $\tau 3$ (times t1 to t3 and t4 to t6) including times t2 and t5 for switching the acoustic gain and the ultrasonic gain, as shown in Fig. 3. The amplification portion 22 is configured to switch the acoustic gain and the ultrasonic gain after the times (t1 and t4) when the imaging portion 23 is switched from a state of acquiring a detection signal to a state of acquiring no detection signal. The signal non-acquiring period $\tau 3$ is smaller than the period (the acoustic period) $\tau 1$ for acquiring the acoustic wave A by applying light from the light source portion 11 to the specimen P.

More specifically, the control portion 21 is configured to transmit a receiving accept signal to the imaging portion 23, as shown in Figs. 1 and 3. For example, the imaging portion 23 is configured to acquire a detection signal from the amplification portion 23 when acquiring a receiving accept signal of a high voltage level. The imaging portion 23 is also configured to acquire no detection signal from the amplification portion 23 when acquiring a receiving accept signal of a low voltage level.

The control portion 21 is also configured to perform control of setting the receiving accept signal to a low voltage level so that the imaging portion 23 acquires no detection signal in a signal non-acquiring period $\tau 3$ including a time t2 for converting the gain control signal from a high voltage level to a low voltage level and a time t5 for converting the gain control signal from a low voltage level to a high voltage level.

The imaging portion 23 is provided with the A-D converter 31. The A-D converter 31 is configured to convert the detection signal (an analog signal) acquired from the amplification portion 23 to a digital signal in correspondence to the sampling trigger signal received from the control portion 21. The A-D converter 31 is connected with a receiving memory 32, and also configured to transmit the detection signal converted to the digital signal to the receiving memory 32.

The imaging portion 23 is also provided with the receiving memory 32. The receiving memory 32 is configured to acquire the receiving accept signal from the control portion 21, and also configured to acquire the detection signal converted to the digital signal from the A-D converter 31 and to temporarily store the same when the receiving accept signal is at a high voltage level. The receiving memory 32 is connected with a data processing portion 33, and also configured to transmit the stored detection signal to the data processing portion 33.

The imaging portion 23 is also provided with the data processing portion 33. The data processing portion 33 is connected with an acoustic image reconstruction portion 34, and configured to transmit data based on the detection signal for the acoustic wave A to the acoustic image reconstruction portion 34. The data processing portion 33 is also connected with an ultrasonic image reconstruction portion 35, and also configured to transmit data based on the detection signal for the ultrasonic wave B2 to the ultrasonic image reconstruction portion 35.

The imaging portion 23 is also provided with the acoustic image reconstruction portion 34. The acoustic image reconstruction portion 34 is configured to perform processing of reconstructing the acquired data based on the detection signal for the acoustic wave A as an image. The acoustic image reconstruction portion 34 is connected with a wave detection/logarithmic converter 36, and also configured to transmit the data based on the detection signal for the acoustic wave A reconstructed as the image to the wave detection/logarithmic converter 36.

The imaging portion 23 is also provided with the wave detection/logarithmic converter 36. The wave detection/logarithmic converter 36 is configured to perform waveform processing of the data reconstructed as the image. The detection/logarithmic converter 36 is connected with an acoustic image construction portion 37, and also configured to transmit the waveform-processed data to the acoustic image construction portion 37.

The imaging portion 23 is also provided with the acoustic image construction portion 37. The acoustic image construction portion 37 is configured to perform processing of constructing a tomographic image in the specimen P on the basis of the waveform-processed data. The acoustic image construction portion 37 is connected with an image synthesis portion 38, and also configured to transmit the tomographic image based on the acoustic wave A to the image synthesis portion 38.

The imaging portion 23 is also provided with the ultrasonic image reconstruction portion 35, another wave detection/logarithmic converter 39 and an ultrasonic image construction portion 40. The ultrasonic image reconstruction portion 35 is configured to perform processing of reconstructing the data based on the detection signal for the ultrasonic wave B2 acquired from the data processing portion 33 as an image. The ultrasonic image reconstruction portion 35 is also configured to transmit a tomographic image based on the ultrasonic wave B2 to the image synthesis portion 38 through the wave detection/logarithmic converter 39 and the ultrasonic image construction portion 40.

The image synthesis portion 38 is configured to perform processing of synthesizing the tomographic images based on the acoustic wave A and the ultrasonic wave B2, and to output a synthetic image to the image display portion 24. In other words, the image synthesis portion 38 images both of the acoustic wave A and the ultrasonic wave B2.

The image display portion 24 includes a liquid crystal panel or the like, and is configured to display the image input therein.

Operations of the photoacoustic imager 100 are now described with reference to Fig. 3. The control portion 21 performs processing related to control of the photoacoustic imager 100.

(Acoustic Period τ1 (before Time t1))

Before the time t1, the control portion 21 inputs the light trigger signal in the light source driving portion 12, so that the light source portion 11 applies light to the specimen P. Further, the control portion 21 inputs a receiving accept signal and a gain control signal of high voltage levels in the imaging portion 23 and the amplification portion 22 respectively. The detection portion 13 detects the acoustic wave A from the specimen P, and transmits a detection signal to the amplification porno 22. The amplification portion 22 having the acoustic gain (about 80 dB) amplifies the detection signal and transmits the amplified detection signal to the imaging portion 23. The imaging portion 23 acquires the amplified detection signal.

(Signal Non-Acquiring Period τ3 (Times t1 to t3))

At the time t1, the control portion 21 converts the receiving accept signal from the high voltage level to a low voltage level, so that the imaging portion 23 stops acquiring the detection signal. At the time t2, the control portion 21 converts the gain control signal from the high voltage level to a low voltage level, and switches the amplification portion 22 from the acoustic gain (about 80 dB) to the ultrasonic gain (about 50 dB).

(Ultrasonic Period τ2 (Times t3 to t4))

At the time t3, the control portion 21 converts the receiving accept signal from the low voltage level to a high voltage level, so that the imaging portion 23 acquires a detection signal amplified by the amplification portion 22 having the ultrasonic gain (about 50 dB).

(Signal Non-Acquiring Period τ3 (Times t4 to t6))

At the time t4, the control portion 21 converts the receiving accept signal from the high voltage level to a low voltage level, so that the imaging portion 23 stops acquiring the detection signal. At the time t5, the control portion 21 converts the gain control signal from the low voltage level to a high voltage level, and switches the amplification portion 22 from the ultrasonic gain (about 50 dB) to the acoustic gain (about 80 dB).

(Acoustic Period τ1 (after Time t6))

After the time t6, the control portion 21 converts the receiving accept signal from the low voltage level to a high voltage level, so that the imaging portion 23 acquires a detection signal amplified by the amplification portion 22 having the acoustic gain (about 80 dB).

According to the first embodiment, the following effects can be attained:

According to the first embodiment, as hereinabove described, the amplification portion 22 is so configured that the acoustic gain corresponding to that of the amplification portion 22 for amplifying the detection signal resulting from the acoustic wave A is larger than the ultrasonic gain corresponding to that of the amplification portion 22 for amplifying the detection signal resulting from the ultrasonic wave B2. Thus, the photoacoustic imager 100 can prevent saturation (excess enlargement) of the detection signal for the ultrasonic wave B2 in the imaging portion 23 while preventing the detection signal for the acoustic wave A from weakening also when employing light-emitting elements (the light-emitting diode elements 11a, for example) having relatively small quantities of light. Consequently, the photoacoustic imager 100 can prevent difficulty in proper imaging of the acoustic wave A and the ultrasonic wave B2 also when the quantity of the light applied to the specimen P is small.

According to the first embodiment, as hereinabove described, the amplification portion 22 is configured to be capable of switching the acoustic gain and the ultrasonic gain. Further, the imaging portion 23 is configured to acquire no detection signal in the signal non-acquiring period τ3 including the time t2 for switching the acoustic gain and the ultrasonic gain. At the time t2 for switching the acoustic gain and the ultrasonic gain, the amplification portion 22 may conceivably erroneously amplify the detection signal resulting from the acoustic wave A with the ultrasonic gain or erroneously amplify the detection signal resulting from the ultrasonic wave B2 with the acoustic gain. In this case, it is conceivable that the detection signal for the acoustic wave A is weakened or the detection signal for the ultrasonic wave B2 is saturated in the imaging portion 23. According to the first embodiment, therefore, the signal non-acquiring period τ3 including the time t2 for switching the acoustic gain and the ultrasonic gain is so provided that the photoacoustic imager 100 can prevent the detection signal for the ultrasonic wave B2 from saturation in the imaging portion 23 while preventing the detection signal for the acoustic wave A from weakening.

According to the first embodiment, as hereinabove described, the light source portion 11 is provided with the light-emitting diode elements 11a. The light-emitting diode elements 11a are lower in directivity as compared with light-emitting elements emitting laser beams, and hence a light emission range remains relatively unchanged also when misregistration takes place. Therefore, the photoacoustic imager 100 requires neither precise alignment (registration) of optical members nor an optical platen or a strong housing for preventing characteristic fluctuation resulting from vibration of an optical system, dissimilarly to a case of employing light-emitting elements emitting laser beams. Consequently, the photoacoustic imager 100 can be prevented from size increase and complication in structure due to the nonrequirement for precise alignment of optical members and an optical platen or a strong housing. Further, each light-emitting diode element 11a has a smaller quantity of light as compared with a light-emitting element emitting a laser beam or the like. According to the first embodiment, therefore, the amplification portion 22 is so configured that the acoustic gain is larger than the ultrasonic gain, whereby the photoacoustic imager 100 can further effectively prevent difficulty in proper imaging of the acoustic wave A and the ultrasonic wave B2.

According to the first embodiment, as hereinabove described, the amplification portion 22 is configured to switch the acoustic gain and the ultrasonic gain after the imaging portion 23 is switched from the state of acquiring the detection signal to the state of acquiring no detection signal. Thus, the photoacoustic imager 100 can more reliably prevent the amplification portion 22 from amplifying the detection signal resulting from the acoustic wave A with the ultrasonic gain or amplifying the detection signal resulting from the ultrasonic wave B2 with the acoustic gain at the times (t1 and t4) for switching the acoustic gain and the ultrasonic gain.

According to the first embodiment, as hereinabove described, the signal non-acquiring period τ3 is smaller than the period (the acoustic period τ1) for acquiring the acoustic wave A by applying light from the light source portion 11 to the specimen P. Thus, the signal non-acquiring period τ3 is smaller than the period for acquiring the acoustic wave A, whereby the period for acquiring the acoustic wave A can be prevented from decreasing also when the signal non-acquiring period τ3 is provided.

According to the first embodiment, as hereinabove described, the light source portion 11 is configured to emit the pulsed light having the wavelength in the infrared region. Thus, the light having the wavelength in the infrared region can relatively easily penetrate a human body, whereby the photoacoustic imager 100 can deliver the light from the light source portion 11 to a deeper portion of the specimen P when the specimen P is prepared from a human body.

According to the first embodiment, as hereinabove described, the amplification portion 22 is provided with the first and second amplifiers 22b and 22e, and so configured that the acoustic gain corresponds to that obtained by synthesizing the gains of the first and second amplifiers 22b and 22e and the ultrasonic gain corresponds to the gain of the second amplifier 22e. Thus, the photoacoustic imager 100 can easily render the acoustic gain larger than the ultrasonic gain.

According to the first embodiment, as hereinabove described, the amplification portion 22 is provided with the switch portion 22c for switching the state corresponding to the gains of the first and second amplifiers 22b and 22e and the state corresponding to the gain of the second amplifier 22e. Thus, the photoacoustic imager 100 can easily switch the acoustic gain and the ultrasonic gain with the switch portion 22.

According to the first embodiment, as hereinabove described, the amplification portion 22 is configured to alternately and repetitively switch the acoustic gain and the ultrasonic gain. Thus, the imaging portion 23 can repetitively acquire the acoustic wave $\underline{A}$ and the ultrasonic wave B2, thereby easily imaging both of the acoustic wave $\underline{A}$ and the ultrasonic wave B2.

According to the first embodiment, as hereinabove described, the period (the acoustic period $\tau1$) when the amplification portion 22 has the acoustic gain is larger than the period (the ultrasonic period $\tau2$) when the amplification portion 22 has the ultrasonic gain. Thus, the period for detecting the acoustic wave $\underline{A}$ is larger than that for detecting the ultrasonic wave B2 also when signal intensity of the acoustic wave $\underline{A}$ is smaller than that of the ultrasonic wave B2, whereby the photoacoustic imager 100 can prevent the detection signal for the ultrasonic wave B2 from saturation in the imaging portion 23 while preventing the detection signal for the acoustic wave $\underline{A}$ from weakening.

(Second Embodiment)

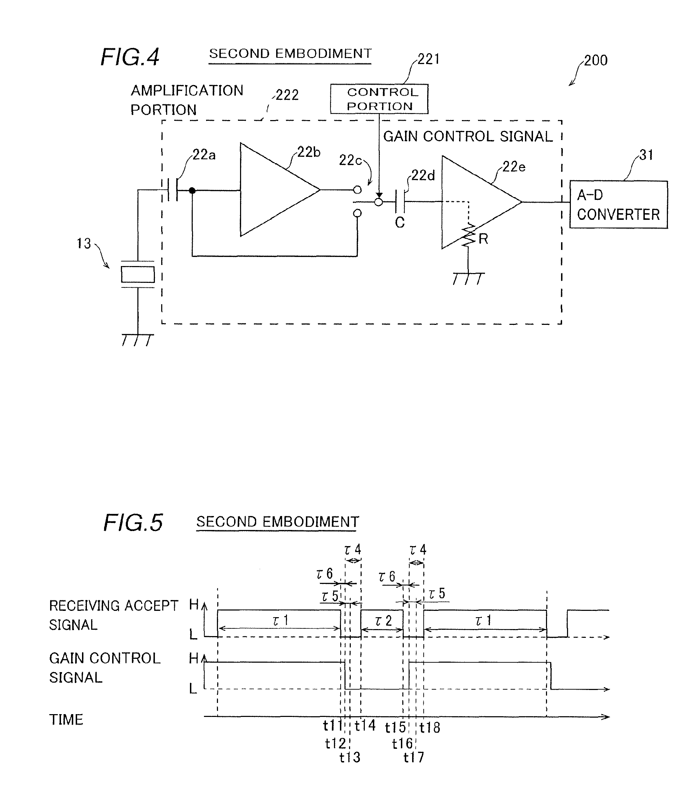

The structure of a photoacoustic imager 200 according to a second embodiment of the present invention is now described with reference to Figs. 4 and 5. According to the second embodiment, the photoacoustic imager 200 is so configured that a signal non-acquiring period $\tau4$ is larger than a time constant $\tau5$ calculated with the capacitance C of a second coupling capacitor 22d and input impedance R of an amplification portion 222.

As shown in Fig. 4, the photoacoustic imager 200 according to the second embodiment is provided with a control portion 221 and the amplification portion 222. The amplification portion 222 is configured similarly to the amplification portion 22 according to the first embodiment, to be capable of switching an acoustic gain and an ultrasonic gain on the basis of a gain control signal received from the control portion 221.

According to the second embodiment, the photoacoustic imager 200 is so configured that the signal non-acquiring period $\tau4$ is larger than the time constant $\tau5$ calculated with the capacitance C of the second coupling capacitor 22d and the input impedance R of a second amplifier 22e (the amplification portion 222), as shown in Fig. 5.

More specifically, the time constant $\tau5$ (times

t12 to t13) is calculated according to the following expression (1), assuming that C represents the capacitance of the second coupling capacitor 22d and R represents the input impedance of the second amplifier 22e:

$$\tau5 = C \times R \ ... \ (1)$$

Assuming that the capacitance C of the second coupling capacitor 22d is 0.1 $\mu$F and the input impedance R of the second amplifier 22e is 10 k$\Omega$, for example, the time constant $\tau5$ is 1 ms. In this case, the control portion 221 controls the amplification portion 222 and an imaging portion 23 so that the signal non-acquiring period $\tau4$ (times t12 to t14) is larger than the time constant $\tau5$.

In other words, the control portion 221 switches a gain control signal from a high voltage level to a low voltage level at the time t12, and thereafter switches a receiving accept signal from a low voltage level to a high voltage level at the time t14 after a lapse of the signal non-acquiring period $\tau4$ larger than the time constant $\tau5$ (times t12 to t13). Further, the control portion 221 switches the gain control signal from the low voltage level to a high voltage level at a time t16, and thereafter switches the receiving accept signal from the low voltage level to a high voltage level at a time t18 after a lapse of the signal non-acquiring period $\tau4$ larger than the time constant $\tau5$ (times t16 to t17).

The control portion 221 is so configured that the photoacoustic imager 200 has another signal non-acquiring period $\tau6$ (times t11 to t12) in addition to the signal non-acquiring period $\tau4$ and the signal non-acquiring period $\tau6$ is larger than zero ($\tau6 > 0$). In other words, the control portion 221 switches the receiving accept signal from a high voltage level to a low voltage level at the time t11, and thereafter switches the gain control signal from a high voltage level to a low voltage level at the time t12 after a lapse of a prescribed period (the signal non-acquiring period) $\tau6$. Further, the control portion 221 switches the receiving accept signal from the high voltage level to a low voltage level at a time t15, and thereafter switches the gain control signal from the low voltage level to a high voltage level after a lapse of the prescribed period (the signal non-acquiring period) $\tau6$. Thus, the amplification portion 222 is reliably prevented from switching the acoustic gain and the ultrasonic gain in an acoustic period $\tau1$ or an ultrasonic period $\tau2$. Further, the time constant $\tau5$ is larger than a time (a settling time) required for settling a factor inhibiting the amplification portion 222 from detecting the acoustic wave $\underline{A}$. In other words, the signal non-acquiring period $\tau4$ is set to be larger than the time (the settling time) required for settling a factor inhibiting the amplification portion 222 from detecting the acoustic wave $\underline{A}$ according to the second embodiment.

The remaining structures of the photoacoustic imager 200 according to the second embodiment are similar to those of the photoacoustic imager 100 according to the first embodiment.

According to the second embodiment, the following effects can be attained:

According to the second embodiment, as hereinabove described, the amplification portion 222 is configured to include the second coupling capacitor 22d for acquiring a detection signal. Further, the photoacoustic imager 200 is so configured that the signal non-acquiring period τ4 is larger than the time constant τ5 calculated with the capacitance C of the second coupling capacitor 22d and the input impedance R of the amplification portion 222 (the second amplifier 22e). The length of the time (the settling time) required for settling a factor, such as a reflected wave or a crosstalk caused during (the times t12 and t16) or immediately after (the times t12 to t13 and t16 to t17) switching of the acoustic gain and the ultrasonic gain, inhibiting the amplification portion 222 from detecting the acoustic wave A and the ultrasonic wave B2 is related to the magnitudes of the capacitance C of the second coupling capacitor 22d of the amplification portion 222 and the input impedance R of the amplification portion 222 (the second amplifier 22e). According to the second embodiment, the photoacoustic imager 200 is so configured that the signal non-acquiring period τ4 is larger than the time constant τ5 calculated with the capacitance C of the second coupling capacitor 22d and the input impedance R of the amplification portion 222 so that the second coupling capacitor 22d acquires no detection signal during the time (the settling time) required for settling the factor inhibiting the amplification portion 222 from detecting the acoustic wave A and the ultrasonic wave B2. Therefore, the photoacoustic imager 200 can be prevented from influence by the factor inhibiting the amplification portion 222 from detecting the acoustic wave A and the ultrasonic wave B2. Consequently, the photoacoustic imager 200 can further reliably prevent the detection signal for the ultrasonic wave B2 from saturation in the imaging portion 23 while preventing the detection signal for the acoustic wave A from weakening.

According to the second embodiment, as hereinabove described, the signal non-acquiring period τ4 is larger than the time required for settling the factor inhibiting the amplification portion 222 from detecting the acoustic wave A. Therefore, the second coupling capacitor 22d does not enter a state of acquiring the detection signal during the settling time required for settling the factor inhibiting the amplification portion 222 from detecting the acoustic wave A and the ultrasonic wave B2, whereby the photoacoustic imager 200 can prevent inhibition of the detection of the acoustic wave A and the ultrasonic wave B2. Consequently, the photoacoustic imager 200 can prevent the detection signal for the acoustic wave A from weakening, and can also prevent the detection signal for the ultrasonic wave B2 from saturation in the imaging portion 23.

The remaining effects of the photoacoustic imager 200 according to the second embodiment are similar to those of the photoacoustic imager 100 according to the first embodiment.

(Third Embodiment)

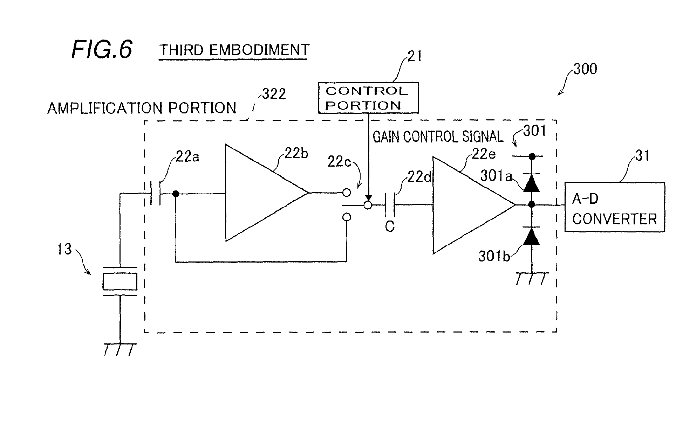

The structure of a photoacoustic imager 300 according to a third embodiment of the present invention is now described with reference to Fig. 6. According to the third embodiment, a clamp circuit 301 is provided on an output side of an amplification portion 322.

As shown in Fig. 6, the photoacoustic imager 300 according to the third embodiment is provided with the amplification portion 322, which in turn is provided with the clamp circuit 301. The clamp circuit 301 is constituted of diodes 301a and 301b.

More specifically, the anode of the diode 301a is connected to an output side of a second amplifier 22e and an input side of an A-D converter 31. The cathode of the diode 301a is connected to a power source portion (not shown), having a prescribed voltage value, of the amplification portion 322. Thus, the amplification portion 322 is so configured that current flows from the output side of the second amplifier 22e to the aforementioned power source portion through the diode 301a when the voltage value on the output side of the second amplifier 22e exceeds a value obtained by adding the forward voltage value of the diode 301a to the prescribed voltage value. In other words, the photoacoustic imager 300 is so configured that the voltage values on the output side of the second amplifier 22e and the input side of the A-D converter 31 are prevented from exceeding the prescribed voltage value. The prescribed voltage value is set to be smaller than a voltage value overshooting (saturating) a detection signal in the A-D converter 31.

The cathode of the diode 301b is connected to the output side of the second amplifier 22e and the input side of the A-D converter 31. The anode of the diode 301a is grounded (connected to the ground). Thus, the amplification portion 322 is so configured that current flows from the ground to the output side of the second amplifier 22e through the diode 301b when the voltage value on the output side of the second amplifier 22e is smaller than substantially zero (a value obtained by subtracting the forward voltage value of the diode 301b from the voltage value of the ground). In other words, the photoacoustic imager 300 is so configured that the voltage values on the output side of the second amplifier 22e and the input side of the A-D converter 31 are prevented from being smaller than substantially zero. It is assumed that the voltage value of substantially zero is larger than a voltage value undershooting the detection signal in the A-D converter 31.

The remaining structures of the photoacoustic imager 300 according to the third embodiment are similar to those of the photoacoustic imager 100 according to the first embodiment.

According to the third embodiment, the following effects can be attained:

According to the third embodiment, as hereinabove described, the clamp circuit 301 is provided on the output side of the amplification portion 322. When the detection signal is saturated in the imaging portion 23 (the A-D converter 31), there generally arises a period when the photoacoustic imager 300 cannot normally acquire the detection signal after the saturation of the detection signal. According to the third embodiment, therefore, the clamp circuit 301 is so provided on the output side of the amplification portion 322 (on the input side of the A-D converter 31) that the photoacoustic imager 300 can restrict the intensity (voltage) of the detection signal output from the amplification portion 322 (input in the A-D converter 31). Thus, the photoacoustic imager 300 can prevent the detection signal from saturation (overshooting) in the A-D converter 31 also when a relatively large detection signal (voltage of a value exceeding the prescribed voltage value) is input in the amplification portion 322 having an acoustic gain, for example. Consequently, the photoacoustic imager 300 can quickly image an acoustic wave A and an ultrasonic wave B2 also when a relatively large detection signal is input in the amplification portion 322 having the acoustic gain.

According to the third embodiment, as hereinabove described, the clamp circuit 301 is provided with the first diode 301a having the anode connected to the output side of the amplification portion 322 and the cathode connected to the power source portion and the second diode 301b having the grounded anode and the cathode connected to the output side of the amplification portion 322. Thus, current flows from the amplification portion 322 to the power source portion when the voltage value on the output side of the amplification portion 322 exceeds the voltage value (the prescribed voltage value) of the power source portion, whereby the photoacoustic imager 300 can prevent the detection signal from saturation (overshooting) with respect to the input side of the imaging portion 23. When the voltage value on the output side of the amplification portion 322 is reduced below zero, on the other hand, the photoacoustic imager 300 can prevent the detection signal from undershooting with respect to the input side of the imaging portion 23.

The remaining effects of the photoacoustic imager 300 according to the third embodiment are similar to those of the photoacoustic imager 100 according to the first embodiment.

(Fourth Embodiment)

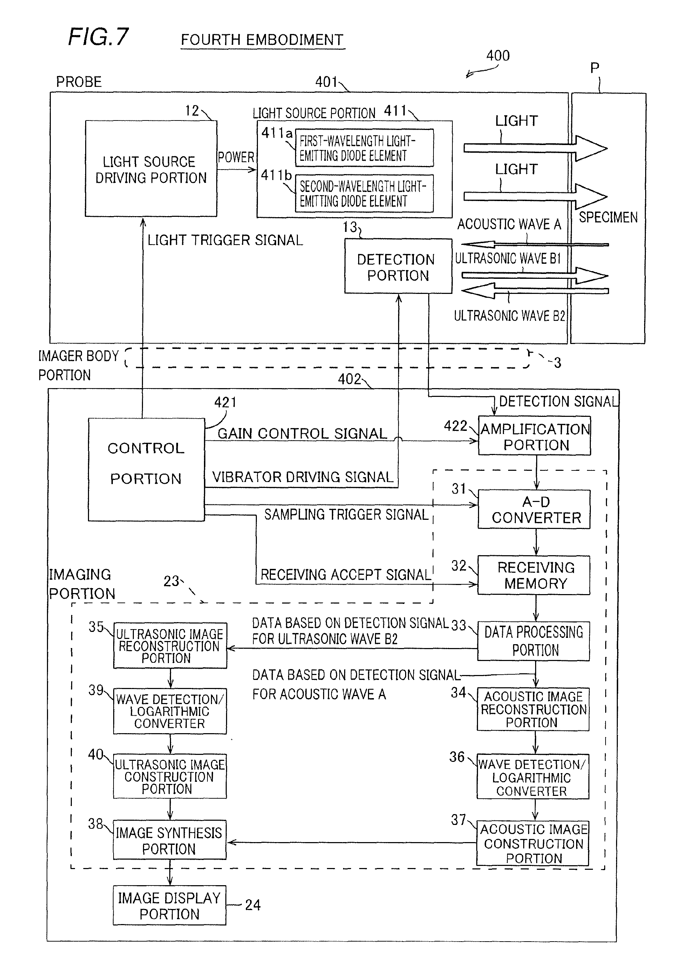

The structure of a photoacoustic imager 400 according to a fourth embodiment of the present invention is now described with reference to Figs. 7 to 9. According to the fourth embodiment, the photoacoustic imager 400 is provided with a first-wavelength light-emitting diode element 411a emitting light having a wavelength of about 850 nm and a second-wavelength light-emitting diode element 411b emitting light having a wavelength of about 760 nm, dissimilarly to the photoacoustic imagers 100, 200 and 300 according to the first to third embodiments provided with only the light-emitting diode elements having a wavelength of about 850 nm.

As shown in Fig. 7, the photoacoustic imager 400 according to the fourth embodiment is provided with a probe 401 and an imager body portion 402. The probe 401 includes a light source portion 411, while the imager body portion 402 includes a control portion 421 and an amplification portion 422.

According to the fourth embodiment, the light source portion 411 includes the first-wavelength light-emitting diode element 411a emitting the light having the wavelength of about 850 nm and the second-wavelength light-emitting diode element 411b emitting the light having the wavelength of about 760 nm. The wavelength of about 850 nm is an example of the "first wavelength" in the present invention. The wavelength of about 760 nm is an example of the "second wavelength" in the present invention. The first-wavelength light-emitting diode element 411a is an example of the "first light-emitting element" in the present invention. The second-wavelength light-emitting diode element 411b is an example of the "second light-emitting element" in the present invention.

According to the fourth embodiment, the amplification portion 422 is so configured that a first acoustic gain which is a gain of the amplification portion 422 for amplifying a detection signal resulting from an acoustic wave A generated due to light from the first-wavelength light-emitting diode element 411a and a second acoustic gain which is a gain of the amplification portion 422 for amplifying a detection signal resulting from an acoustic wave A generated due to light from the second-wavelength light-emitting diode element 411b have values different from each other, while the first and second acoustic gains are set to be larger than an ultrasonic gain.

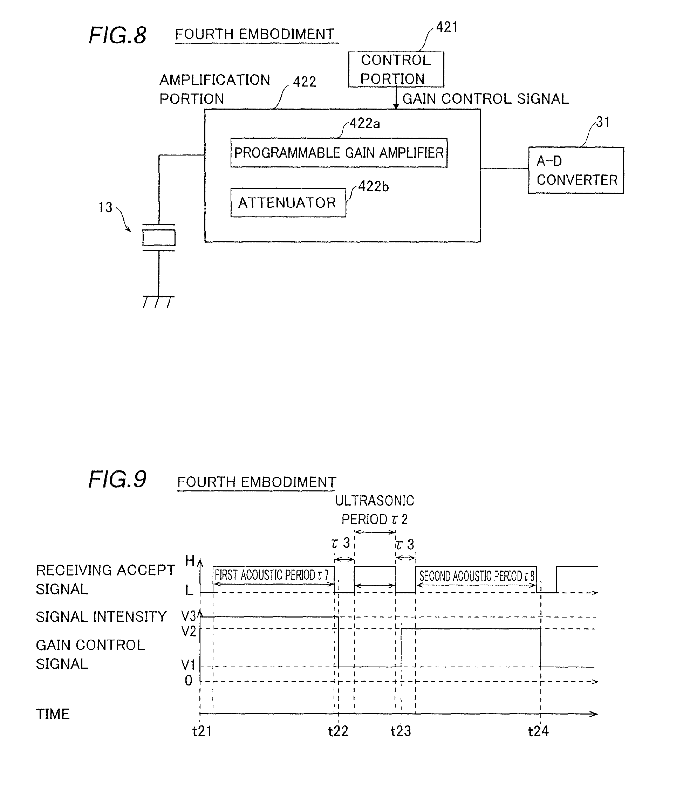

More specifically, the amplification portion 422 includes a programmable gain amplifier 422a and an attenuator 422b, as shown in Fig. 8. The control portion 421 is configured to control the gain of the amplification portion 422 with a gain control signal constituted of an analog signal. In this case, the gain control signal is so constituted of the analog signal as to prevent influence by an electromagnetic wave or the like (noise) as compared with a case of constituting the gain control signal of a digital signal.

As shown in Fig. 9, the control portion 421 is configured to provide signal non-acquiring periods $\tau 3$ between the respective ones of a period (a first acoustic period $\tau 7$) for acquiring a detection signal by applying light from the first-wavelength light-emitting diode element 411a to a specimen P, a period (a second acoustic period $\tau 8$) for acquiring a detection signal by applying light from the second-wavelength light-emitting diode element 411b to the specimen P and an ultrasonic period

$\tau$2.

The control portion 421 is also configured to set the gain control signal to a voltage level V3 in a period (times t21 to t22) including the first acoustic period $\tau$7, to a voltage level V2 in a period (times t23 to t24) including the second acoustic period $\tau$8 and to a voltage level V1 in a period (times t22 to t23) including the ultrasonic period $\tau$2. Thus, the control portion 421 is configured to have the first acoustic gain in the first acoustic period $\tau$7, to have the second acoustic gain in the second acoustic period $\tau$8 and to have the ultrasonic gain in the ultrasonic period $\tau$2 with the programmable gain amplifier 422a and the attenuator 422b on the basis of the gain control signal.

The amplification portion 422 is so configured that the first acoustic gain is larger than the second acoustic gain and the second acoustic gain is larger than the ultrasonic gain. The amplification portion 422 is also configured to be capable of rendering the first acoustic gain smaller than the second acoustic gain on the basis of the gain control signal received from the control portion 421. Thus, the amplification portion 422 is configured to be capable of changing a case of rendering the first acoustic gain larger than the second acoustic gain and a case of rendering the former smaller than the latter in response to an absorption coefficient of a detection object in the specimen P corresponding to each wavelength.

The remaining structures of the photoacoustic imager 400 according to the fourth embodiment are similar to those of the photoacoustic imager 100 according to the first embodiment.

According to the fourth embodiment, the following effects can be attained:

According to the fourth embodiment, as hereinabove described, the light source portion 411 is configured to include the first-wavelength light-emitting diode element 411a emitting the light having the wavelength of about 850 nm and the second-wavelength light-emitting diode element 411b emitting the light having the wavelength of about 760 nm. Further, the amplification portion 422 is so configured that the first acoustic gain which is the gain of the amplification portion 422 for amplifying the detection signal resulting from the acoustic wave A generated due to light from the first-wavelength light-emitting diode element 411a and the second acoustic gain which is the gain of the amplification portion 422 for amplifying the detection signal resulting from the acoustic wave A generated due to light from the second-wavelength light-emitting diode element 411b have values different from each other. In addition, the amplification portion 422 is also so configured that the first and second acoustic gains are larger than the ultrasonic gain. Thus, the photoacoustic imager 400 can prevent the detection signal resulting from the acoustic wave A generated due to light from the second-wavelength light-emitting diode element 411b and a detection signal resulting from an ultrasonic wave B2 from excess enlargement while preventing the detection signal resulting from the acoustic wave A generated due to light from the first-wavelength light-emitting diode element 411a from weakening by rendering the first acoustic gain larger than the second acoustic gain when the specimen P has a relatively small absorption coefficient for the light having the wavelength of about 850 nm and a relatively large absorption coefficient for the light having the wavelength of about 760 nm, for example.

According to the fourth embodiment, as hereinabove described, the amplification portion 422 is provided with the programmable gain amplifier 422a and the attenuator 422b. Thus, the programmable gain amplifier 422a and the attenuator 422b can control gains with the gain control signal constituted of the analog signal, whereby the photoacoustic imager 400 can suppress influence by an electromagnetic wave or the like (noise) resulting from a control signal as compared with a case of controlling gains of a plurality of amplifiers by supplying a digital signal to a switch portion.

According to the fourth embodiment, as hereinabove described, the control portion 421 is configured to transmit the gain control signal consisting of the analog signal to the amplification portion 422, which in turn is configured to switch the acoustic gain and the ultrasonic gain with the programmable gain amplifier 422a and the attenuator 422b on the basis of the gain control signal received from the control portion 421. Thus, the amplification portion 422 can switch the acoustic gain and the ultrasonic gain with the gain control signal consisting of the analog signal, whereby the same can easily switch the acoustic gain and the ultrasonic gain while preventing influence by an electromagnetic wave or the like (noise) resulting from a control signal.

The remaining effects of the photoacoustic imager 400 according to the fourth embodiment are similar to those of the photoacoustic imager 100 according to the first embodiment.

Although the present invention has been described and illustrated in detail, it is clearly understood that the same is by way of illustration and example only and is not to be taken by way of limitation, the spirit and scope of the present invention being limited only by the terms of the appended claims.

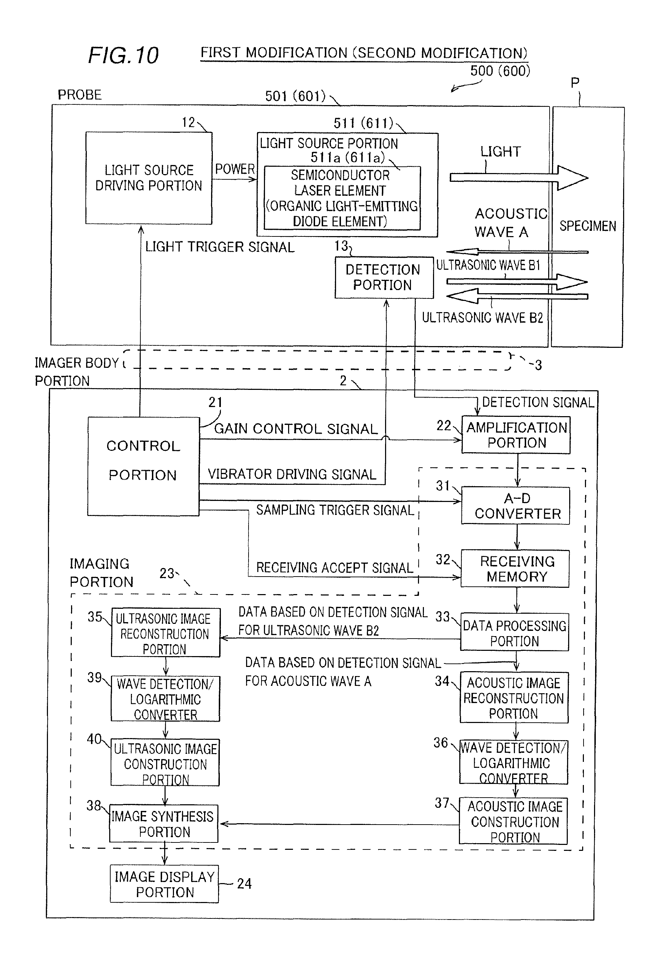

For example, while the light-emitting diode elements are employed as light-emitting elements in each of the aforementioned first to fourth embodiments, the present invention is not restricted to this. According to the present invention, light-emitting elements other than the light-emitting diode elements may alternatively be employed. For example, semiconductor laser elements 511a or organic light-emitting diode elements 611a may be employed as light-emitting elements, as in a first or second modification shown in Fig. 10.

A photoacoustic imager 500 according to the first modification is provided with a probe 501, as shown in Fig. 10. The probe 501 includes a light source portion 511, which in turn includes the semiconductor laser elements 511a. The semiconductor laser elements 511a are configured to be capable of applying light to a specimen P. In this case, the semiconductor laser elements 511a can apply laser beams relatively higher in directivity as compared with light-emitting diode elements to the specimen P, whereby the photoacoustic imager 500 can reliably apply most part of the light from the semiconductor laser elements 511a to the specimen P.

A photoacoustic imager 600 according to the second modification is provided with a probe 601, as shown in Fig. 10. The probe 601 includes a light source portion 611, which in turn includes the organic light-emitting diode elements 611a. The organic light-emitting diode elements 611a are configured to be capable of applying light to a specimen P. In this case, the organic light-emitting diode elements 611a are easily reducible in thickness, whereby the light source portion 611 can be easily miniaturized.

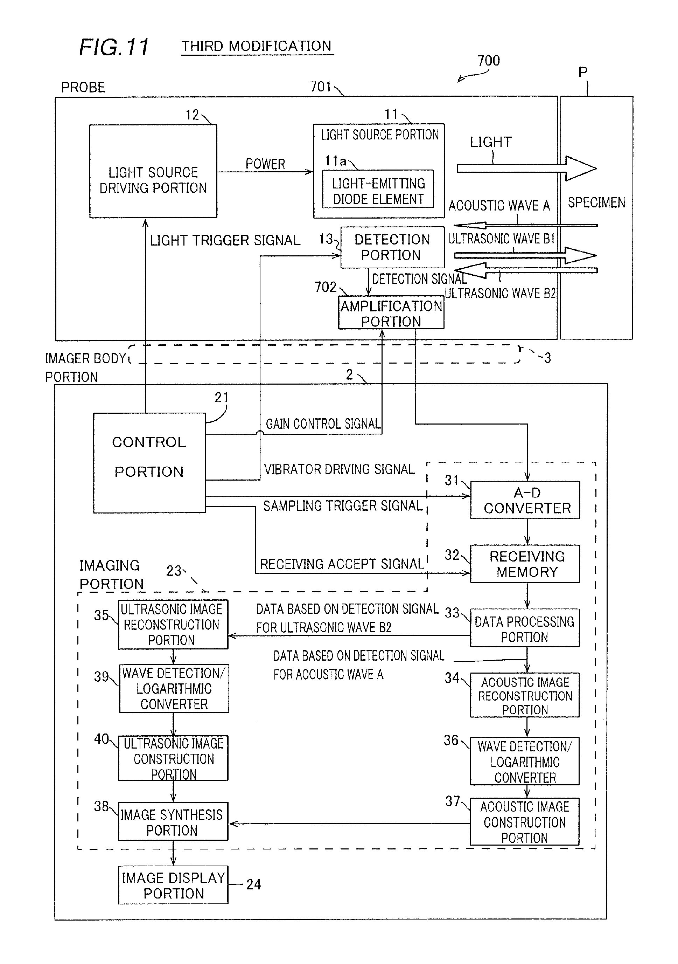

While the photoacoustic imager is so configured that the amplification portion is provided on the imager body portion in each of the aforementioned first to fourth embodiments, the present invention is not restricted to this. According to the present invention, the amplification portion may alternatively be provided on an element other than the imager body portion. For example, an amplification portion 702 may be provided on a probe 701, as in a third modification shown in Fig. 11.

A photoacoustic imager 700 according to the third modification includes the probe 701, which in turn includes the amplification portion 702, a light source portion 11 and a detection portion 13. The amplification portion 702 is configured to acquire a detection signal from the detection portion 13, to amplify the detection signal and to transmit the amplified detection signal to an imaging portion 23 of an imager body portion 2 through a cable 3.

When a signal transmitted through the cable 3 has a relatively high voltage value, influence by an electromagnetic wave or the like (noise) from outside the cable 3 is suppressed. The detection signal output from the detection portion 13 has a voltage value on the order of $\mu$V. The amplification portion 702 so amplifies the voltage that the voltage output therefrom reaches a value on the order of mV to about several V, whereby influence by an electromagnetic wave or the like (noise) from outside the cable 3 is suppressed when the detection signal is transmitted through the cable 3 due to the aforementioned structure according to the third modification.

While the amplification portion is configured to switch the acoustic gain and the ultrasonic gain in each of the aforementioned first to fourth embodiments, the present invention is not restricted to this. According to the present invention, the amplification portion may alternatively be configured not to switch the acoustic gain and the ultrasonic gain. For example, the photoacoustic imager may be provided with two amplification portions in total, i.e., an amplification having an acoustic gain and that having an ultrasonic gain.

While the amplification portion is provided with the plurality of (first and second) amplifiers and configured to have the acoustic and ultrasonic gains in each of the aforementioned first to third embodiments, the present invention is not restricted to this. According to the present invention, the amplification portion may alternatively be provided with either an amplifier or an attenuator, and configured to have acoustic and ultrasonic gains.

While the amplification portion is provided with the programmable gain amplifier and the attenuator and configured to have the first and second acoustic gains and the ultrasonic gain in the aforementioned fourth embodiment, the present invention is not restricted to this. According to the present invention, the amplification portion may alternatively be provided with either a programmable gain amplifier or an attenuator and configured to have first and second acoustic gains and an ultrasonic gain.

While the acoustic and ultrasonic gains are set to 80 dB and 50 dB respectively in each of the aforementioned first to fourth embodiments, the present invention is not restricted to this. According to the present invention, the acoustic and ultrasonic gains may alternatively be set to values other than 80 dB and 50 dB. In other words, the acoustic gain may simply be larger than the ultrasonic gain according to the present invention.

While the light-emitting diode elements emitting the light having the wavelength of about 850 nm or about 760 nm are employed in each of the aforementioned first to fourth embodiments, the present invention is not restricted to this. According to the present invention, light-emitting elements emitting light having a wavelength other than about 850 nm or about 760 nm may alternatively be employed.

## Claims

1. A photoacoustic imager (100, 200, 300, 400, 500, 600, 700) comprising:

 a light-emitting element (11a, 411a, 411b, 511a, 611a) applying light to a specimen;
 a detection portion (13) configured to apply an ultrasonic wave to the specimen and to generate a detection signal by detecting the ultrasonic wave reflected by a detection object in the specimen and an acoustic wave generated by the detection object in the specimen absorbing the light applied from the light-emitting element to the specimen;
 an imaging portion (23) configured to perform imaging on the basis of the detection signal; and
 an amplification portion (22, 222, 322, 422, 702) configured to amplify the detection signal generated by the detection portion and to transmit the detection signal to the imaging portion, wherein
the amplification portion is so configured that an acoustic gain which is a gain of the amplification portion for amplifying the detection signal resulting from the acoustic wave is larger than an ultrasonic gain which is a gain of the amplification portion for amplifying the detection signal resulting from the ultrasonic wave.

2. The photoacoustic imager according to claim 1, wherein
the amplification portion is configured to switch the acoustic gain and the ultrasonic gain, and
the imaging portion is configured not to acquire the detection signal in a signal non-acquiring period including a time for switching the acoustic gain and the ultrasonic gain.

3. The photoacoustic imager according to claim 2, wherein
the amplification portion is configured to switch the acoustic gain and the ultrasonic gain after switching the imaging portion from a state of acquiring the detection signal to a state of not acquiring the detection signal.

4. The photoacoustic imager according to claim 2 or 3, wherein
the amplification portion includes a coupling capacitor (22d) for acquiring the detection signal, and
the signal non-acquiring period is larger than a time constant calculated with the capacitance of the coupling capacitor and the input impedance of the amplification portion.

5. The photoacoustic imager according to any of claims 2 to 4, wherein
the signal non-acquiring period is larger than a time required for settling a factor inhibiting the amplification portion from detecting the acoustic wave.

6. The photoacoustic imager according to any of claims 2 to 5, wherein
the signal non-acquiring period is smaller than a period for acquiring the acoustic wave by applying the light from the light source portion to the specimen.

7. The photoacoustic imager according to any of claims 1 to 6, wherein
a clamp circuit (301) is provided on an output side of the amplification portion.

8. The photoacoustic imager according to any of claims 1 to 7, wherein
the light-emitting element includes a first light-emitting element (411a) emitting light having a first wavelength and a second light-emitting element (411b) emitting light having a second wavelength, and
the amplification portion is so configured that a first acoustic gain which is a gain of the amplification portion for amplifying the detection signal resulting from the acoustic wave generated due to the light from the first light-emitting element and a second acoustic gain which is a gain of the amplification portion for amplifying the detection signal resulting from the acoustic wave generated due to the light from the second light-emitting element have values different from each other and also so configured that the first acoustic gain and the second acoustic gain are larger than the ultrasonic gain.

9. The photoacoustic imager according to any of claims 1 to 8, wherein
the light-emitting element is constituted of a light-emitting diode element (11a, 411a, 411b).

10. The photoacoustic imager according to any of claims 1 to 9, wherein
the light-emitting element is constituted of a semiconductor laser element (511a).

11. The photoacoustic imager according to any of claims 1 to 10, wherein
the light-emitting element is configured to emit pulsed light having a wavelength in the infrared region.

12. The photoacoustic imager according to any of claims 1 to 11, wherein
the amplification portion includes a first amplifier (22b) having a first prescribed gain and a second amplifier (22e) having a second prescribed gain,
the acoustic gain is a gain obtained by synthesizing the gain of the first amplifier and the gain of the second amplifier, and
the ultrasonic gain is the gain of the second amplifier.

13. The photoacoustic imager according to claim 12, wherein
the amplification portion includes a switch portion (22c) for switching a state corresponding to the gain of the first amplifier and the gain of the second amplifier and a state corresponding to the gain of the second amplifier.

14. The photoacoustic imager according to any of claims 1 to 11, wherein
the amplification portion includes at least a programmable gain amplifier (422a) in either the programmable gain amplifier or an attenuator (422b).

15. The photoacoustic imager according to claim 14, further comprising a control portion (421) transmitting a gain control signal consisting of an analog signal to the amplification portion, wherein
the amplification portion is configured to switch the acoustic gain and the ultrasonic gain with at least the programmable gain amplifier in either the programmable gain amplifier or the attenuator.

EP 2 998 735 A1

FIG.1    FIRST EMBODIMENT

*FIG.2*   FIRST EMBODIMENT

CONTROL
PORTION ⟋21

AMPLIFICATION PORTION ⟋22

GAIN CONTROL SIGNAL

22a   22b   22c   22d   22e

A-D
CONVERTER ⟋31

13

*FIG.3*

FIRST EMBODIMENT

ULTRASONIC PERIOD $\tau 2$

ACOUSTIC
PERIOD $\tau 1$   $\tau 3$   $\tau 3$   ACOUSTIC
PERIOD $\tau 1$

RECEIVING ACCEPT
SIGNAL   H   L

GAIN CONTROL
SIGNAL   H   L

TIME

t1   t3   t4   t6
t2   t5

*FIG.4*    SECOND EMBODIMENT

AMPLIFICATION PORTION

CONTROL PORTION /221

/222

/200

GAIN CONTROL SIGNAL

22a  22b  22c  22d  22e  /31

13

A-D CONVERTER

C    R

*FIG.5*    SECOND EMBODIMENT

RECEIVING ACCEPT SIGNAL

GAIN CONTROL SIGNAL

TIME

τ4  τ4
τ6  τ6  τ5
τ5
τ1  τ2  τ1

t11  t14  t15  t18
t12  t16
t13  t17

19

*FIG.6*   THIRD EMBODIMENT

*FIG.7*      FOURTH EMBODIMENT

*FIG.8*    FOURTH EMBODIMENT

421

CONTROL
PORTION

AMPLIFICATION   422
PORTION

GAIN CONTROL SIGNAL

422a

PROGRAMMABLE GAIN AMPLIFIER

422b

ATTENUATOR

31

A-D
CONVERTER

13

*FIG.9*    FOURTH EMBODIMENT

ULTRASONIC
PERIOD τ 2

τ 3     τ 3

RECEIVING ACCEPT
SIGNAL

H

FIRST ACOUSTIC PERIOD τ 7     SECOND ACOUSTIC PERIOD τ 8

L

SIGNAL INTENSITY   V3

GAIN CONTROL
SIGNAL

V2

V1

0

TIME

t21      t22   t23       t24

FIG.10    FIRST MODIFICATION (SECOND MODIFICATION)
                                                    500 (600)

PROBE                          501 (601)                              P

LIGHT SOURCE
DRIVING PORTION    POWER    LIGHT SOURCE PORTION
    12                      511 (611)
                           511a (611a)
                           SEMICONDUCTOR
                           LASER ELEMENT
                           (ORGANIC LIGHT-EMITTING
                           DIODE ELEMENT)

LIGHT

ACOUSTIC
WAVE A    SPECIMEN

LIGHT TRIGGER SIGNAL    13
                           DETECTION
                           PORTION    ULTRASONIC WAVE B1

                                       ULTRASONIC WAVE B2

IMAGER BODY
PORTION                         2                          3

                                       DETECTION SIGNAL

CONTROL
PORTION
    21      GAIN CONTROL SIGNAL    22    AMPLIFICATION
                                         PORTION

            VIBRATOR DRIVING SIGNAL
                                    31    A-D
            SAMPLING TRIGGER SIGNAL        CONVERTER

IMAGING
PORTION    23    RECEIVING ACCEPT SIGNAL    32    RECEIVING
                                                  MEMORY

35    ULTRASONIC IMAGE    DATA BASED ON DETECTION SIGNAL    33
      RECONSTRUCTION      FOR ULTRASONIC WAVE B2              DATA PROCESSING
      PORTION                                                 PORTION

39    WAVE DETECTION/    DATA BASED ON DETECTION SIGNAL
      LOGARITHMIC        FOR ACOUSTIC WAVE A    34    ACOUSTIC IMAGE
      CONVERTER                                       RECONSTRUCTION
                                                      PORTION

40    ULTRASONIC IMAGE
      CONSTRUCTION                                36    WAVE DETECTION/
      PORTION                                           LOGARITHMIC
                                                        CONVERTER

38    IMAGE SYNTHESIS                             37    ACOUSTIC IMAGE
      PORTION                                           CONSTRUCTION
                                                        PORTION

      IMAGE DISPLAY
      PORTION    24

*FIG.11* <u>THIRD MODIFICATION</u>

Europäisches
Patentamt
European
Patent Office
Office européen
des brevets

# EUROPEAN SEARCH REPORT

**Application Number**

EP 15 18 0502

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 6 979 292 B2 (KANAYAMA SHOICHI [JP] ET AL) 27 December 2005 (2005-12-27)<br>* abstract *<br>* column 8, line 11 - line 47 *<br>* column 13, line 43 - line 45 *<br>* figures 1, 2 * | 1,8-11, 14,15<br>7 | INV.<br>G01N29/24<br>A61B5/00<br>G01S15/89<br>G01N21/17 |
| A | US 2011/230750 A1 (TATEYAMA JIRO [JP]) 22 September 2011 (2011-09-22)<br>* abstract *<br>* paragraph [0012] - paragraph [0013] *<br>* figure 1 * | 1 | |
| Y | US 2007/055155 A1 (OWEN NEIL [US] ET AL) 8 March 2007 (2007-03-08)<br>* abstract *<br>* paragraph [0059] *<br>* figure 6E * | 7 | |
| X,P | WO 2014/178257 A1 (FUJIFILM CORP [JP]) 6 November 2014 (2014-11-06)<br>* abstract *<br>* figure 2 * | 1 | TECHNICAL FIELDS SEARCHED (IPC)<br><br>G01N<br>A61B<br>G01S |

The present search report has been drawn up for all claims

| Place of search | Date of completion of the search | Examiner |
|---|---|---|
| Munich | 18 January 2016 | Rouault, Patrick |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 15 18 0502

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2016

| Patent document cited in search report | | Publication date | Patent family member(s) | | Publication date |
|---|---|---|---|---|---|
| US 6979292 | B2 | 27-12-2005 | CA | 2435990 A1 | 02-01-2005 |
| | | | CN | 1575770 A | 09-02-2005 |
| | | | EP | 1493380 A1 | 05-01-2005 |
| | | | JP | 4406226 B2 | 27-01-2010 |
| | | | JP | 2005021380 A | 27-01-2005 |
| | | | KR | 20050003948 A | 12-01-2005 |
| | | | KR | 20060080562 A | 10-07-2006 |
| | | | US | 2005004458 A1 | 06-01-2005 |
| US 2011230750 | A1 | 22-09-2011 | JP | 5393552 B2 | 22-01-2014 |
| | | | JP | 2011194013 A | 06-10-2011 |
| | | | US | 2011230750 A1 | 22-09-2011 |
| US 2007055155 | A1 | 08-03-2007 | NONE | | |
| WO 2014178257 | A1 | 06-11-2014 | JP | 2014213158 A | 17-11-2014 |
| | | | WO | 2014178257 A1 | 06-11-2014 |

For more details about this annex : see Official Journal of the European Patent Office, No. 12/82

**REFERENCES CITED IN THE DESCRIPTION**

**Patent documents cited in the description**

- JP 2012196308 A